# EUROPEAN PATENT SPECIFICATION

(11) **EP 1 385 005 B1**
(45) Date of publication and mention of the grant of the patent: **17.01.2007**
(21) Application number: 02017004.9
(22) Date of filing: 27.07.2002
(51) Int. Cl.: G01N 33/573, G01N 33/574, C12Q 1/48

(54) **Method for determination of thymidine kinase 1 activity and the use thereof**
Verfahren zur Bestimmung der Aktivität von Thymidinkinase-1 und dessen Verwendung
Procédé pour la determination d'activité de thymidine kinase 1 et leur utilisation

(43) Date of publication of application: 28.01.2004
(73) Proprietor: DiaSorin AB, 161 02 Bromma (SE)
(72) Inventor: Oehrvik, Anders, 16102 Bromma (SE); Eriksson, Staffan, 16102 Bromma (SE)
(74) Representative: Capasso, Olga

(56) References cited:
- EP-A- 0 094 923
- WO-A-95/04758
- GOUJON L ET AL: "Monitoring of intracellular levels of 5'-monophosphate-AZT using an enzyme immunoassay" JOURNAL OF IMMUNOLOGICAL METHODS, ELSEVIER SCIENCE PUBLISHERS B.V.,AMSTERDAM, NL, vol. 218, no. 1-2, 1 September 1998 (1998-09-01), pages 19-30, XP004146529 ISSN: 0022-1759
- DATABASE BIOSIS [Online] BIOSCIENCES INFORMATION SERVICE, PHILADELPHIA, PA, US; January 2001 (2001-01) D'CRUZ OSMOND J ET AL: "Thymidine kinase-independent intracellular delivery of bioactive nucleotides by aryl phosphate derivatives of bromo-methoxy zidovudine (compounds WHI-05 and WHI-07) in normal human female genital tract epithelial cells and sperm." Database accession no. PREV200100099778 XP002225459 & BIOLOGY OF REPRODUCTION, vol. 64, no. 1, January 2001 (2001-01), pages 51-59, ISSN: 0006-3363
- KUROIWA NAMIKO ET AL: "Specific recognition of cytosolic thymidine kinase in the human lung tumor by monoclonal antibodies raised against recombinant human thymidine kinase." JOURNAL OF IMMUNOLOGICAL METHODS, vol. 253, no. 1-2, 2001, pages 1-11, XP002225458 ISSN: 0022-1759

## Description

### Technical field

The present invention concerns a new method that enables rapid, accurate and specific determination of thymidine kinase 1 activity in human or animal body fluids and tissue/cell extracts for routine analysis of samples with low levels of activity. The invention further relates to the use of said method for the diagnosis, monitoring and prognostics of diseases such as cancer.

### Background of the invention

In clinical practice tumour diagnosis is of an enormous relevance. Some parameters, e.g. the relative proportion of S phase cells (i.e. DNA synthesising cells) in tumours - as a mean to measure the proliferation rate of tumour cells, have been shown to be very valuable for tumour diagnosis, as well as in monitoring and prognosis of tumour disease.

Earlier methods were classically based on incorporation of an isotopically labelled DNA precursor, e.g. thymidine, 5-lodo-deoxyuridine, 5-Bromodeoxyuridine (1). Another relevant method known in the art is the flow cytometric determination of the amount of DNA per cell requiring a relative large number of cells and involving a complex procedure and expensive instrumentation. In addition, immunological methods for quantification of enzyme/protein markers are used. Said markers comprising Ki67 (MIB)-antigen and PCNA (2) - proteins found in S-phase and G2-phase cells but also to some extent in G1-phase cells are known to be principally expressed in proliferating cells. Unfortunately, these antigen based methods have a lack of specificity for DNA synthesising cells as well as a limited application as these antigens do not occur in body fluids.

Thymidine kinase 1 (TK1, ATP:thymidine 5'-phosphotransferase) is a cytoplasmic enzyme that is only found in proliferating cells. TK1 transfers a γ-phosphate group from a nucleoside triphosphate to the 5'-hydroxyl group of thymidine or deoxyuridine and the resulting products are subsequently phosphorylated to DNA precursors (3-13). TK1 activity starts in late G1-phase, increases in S-phase coinciding with the increase in DNA synthesis and disappears during mitosis (4, 11-13). The variation in TK1 activity is partly due to S-phase regulation of the TK1 promoter as reviewed by Wintersberger (13) but also post-translational mechanisms are important for the level of functional TK1.

As the enzyme shows a restrictive expression pattern being expressed during S-phase of a cell cycle (3-4), TK1 has been used as a reliable and sensitive marker for quantifying the number of S-phase cells in a sample. The activity of TK1 is a prerequisite for methods based on incorporation of isotopes into DNA.

TK1 also phosphorylates derivatives of thymidine or deoxyuridine (dUrd). In particular, TK1 can phosphorylate analogs with modifications at the 5'-position of the pyrimidine ring, and the 3'-position of the ribose (6-9), e.g. 3'-fluoro-3'-deoxythymidin (Aluvidine), 3'-azido-3'-deoxythymidine (Zidovudine, AZT, an anti-HIV compound) (6-8) and 5-substituted dUrd analogs such as 5-fluoro dUrd, 5-bromo dUrd, and 5-ethyl dUrd.

Eukaryotic cells also express a second enzyme phosphorylating thymidine and deoxyuridine, i.e. the mitochondrial thymidine kinase TK2 (15, 16), which enzyme complicates a method for measuring TK activity in S-phase cells.

TK2 having a role in mitochondrial DNA synthesis (16) differs in amino acid sequence, cellular localization - the protein is transported into mitochondria (15) - and in expression profile from TK1. Also the substrate specificity of TK2 differs from that of TK1, as for example the thymidine analogue AZT is phosphorylated by TK2 at significantly lower level than by TK1 (6, 7). A number of dUrd analogues are phosphorylated by TK2 such as 5-IododUrd, but also deoxycytidine and related analogues (6, 7) are phosphorylated. In summary, TK2 plays an essential role for the synthesis of mitochondrial DNA precursors and is involved in certain forms of mitochondrial diseases but not in diseases related to cell proliferation.

Serum TK levels have been determined using a commercially available and highly sensitive ¹²⁵I-dUrd assay (TK-REA). This assay has a proven role in cancer diagnostics, provide prognostic information for relapse free survival as well as over all survival and in some cases aid the choice of therapy (17-24). Recently, high TK serum levels have been used to identify a subgroup of patient having a high risk of disease progression in chronic lymphocytic leukaemia (18). In a series of studies from France monitoring a large number of breast cancer patients, it was demonstrated that cytosolic TK1 levels were the best predictive marker for progression of said disease (20-24). Although the usefulness of the present commercial assay is unquestionable, it has certain disadvantages relating to the use of I¹²⁵, i.e. it is time consuming and is relatively complex to handle. Secondly, said assay does not differentiate between TK1 and TK2 and consequently measures also the TK2 activity in a sample resulting in some cases in false positive results. Thirdly, the presence of thymidine phosphatase - which can be found in serum samples and which occur at varying levels in tumour diseases (27, 28) - and uridine phosphorylase may lead to false negative results in the TK-REA assay, as these enzymes degrade ¹²⁵I-dUrd to deoxyribose and base.

A series of recent publications describe the production and use of antibodies against TK1 (28-35). Unfortunately, immunologically methods based on TK1 levels lack of sensitivity compared to enzyme-based assay systems making it unlikely that an immunologic assay can be used to determine the low levels of TK1 as found in normal subjects. As a second problem, TK1-antibodies react with the active (native) and inactive (degraded) form of TK1. Furthermore, naturally occurring modifications of TK1 may alter the reactivity with different TK1-antibodies. It has been described that TK1 can be found in serum in an active stable multimeric form (36), which form probably originates from disrupted proliferating tumour cells. It is likely that several epitopes of TK1 are masked within this conformational form altering the quality of antibody-based assays.

One problem concerning the enzyme-based assays presently used is the need for high concentrations (mM) of the phosphate donor ATP in order to get a maximum rate of the reaction. In addition, said methods are relatively complex to handle as reaction products have to be separated from its educts which sometimes generates unsatisfactory results (17).

Therefore, there is a definite need for an enzyme-based method to specifically determine TK1 which method should be based on a non-radiometric technique, and should be fast, safe and reproducible.

### Description of the invention

Surprisingly it has now been found that a method to determine TK1 activity in a sample may be fast, safe and reproducible by using a 3'-derivative of thymidine as a substrate for TK1 and measuring the amount of 5'-phosphorylated 3'-derivative of thymidine which is related to the TK1 activity.

In a first embodiment of this invention there is provided a method for determining thymidine kinase 1 activity in a human or animal body fluid or cell or tissue sample, comprising the steps of reacting said sample with a substrate for said thymidine kinase 1 which substrate is a 3'-derivative of thymidine in the presence of a phosphate donor and a buffer system and determining the amount of 5'-phosphorylated 3'-derivative of thymidine formed said amount being related to said thymidine kinase 1 activity.

In connection with the invention thymidine kinase 1 (TK1) refers to the enzyme known as ATP:thymidine 5'-phosphotransferase.

Human or animal body fluid sample in connection with the invention preferably refers to serum and/or plasma samples.

According to this invention, cell sample preferably refers to a cell sample comprising cytosol.

In accordance to this invention, tissue sample refers to solid tissue biopsies.

Phosphate donor in connection with this invention refers to a nucleoside triphosphate suitable to transfer a phosphate group to a substrate. In particular, phosphate donor in connection with this invention refers to adenosine triposphate (ATP) or cytidine triphosphate (CTP), whereby ATP is preferred.

According to this invention, buffer system refers to a buffer suitable to carry out a method as disclosed herein. Expediently, a buffer comprises at least Dithioerythritol (DTE), ATP, MgCl₂ and HEPES or Tris. In particular, a buffer comprises 10-100 mM HEPES or Tris with pH ranging from 6.8-8.0, 1-30 mM DTE, 0,2-8 mM ATP and MgCl₂ at a concentration of at least two times the concentration of ATP. Preferably, a buffer is chosen that does not compete for Mg²⁺ binding with ATP.

According to this invention, substrate for thymidine kinase 1 (TK1) refers to a 3'-derivative of thymidine. In particular it refers to a 3'-deoxy-thymidine derivative of formula I 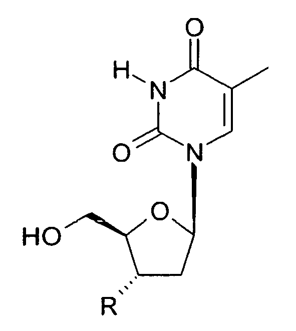 in which R is selected from but not limited to the group consisting of NH₂, NHCOCH₃, SC₂H₅, OC₂H₅, OBn, N₃, NO₂, OCOCH₃,OSO₂CH₃ and F.

It is particularly preferred that a 3'-derivative of thymidine is selected from the group of 3'-azido-3'-deoxythymidine (AZT, INN: Zidovudine) and 3'-Fluoro-3'deoxythymidine (INN: Alovudine).

According to this invention, phosphorylated 3'-derivative of thymidine refers to a 5'-phophorylated 3'-derivative of thymidine. AZTMP (5'-monophosphate of AZT) is a preferred 5'-phophorylated 3'-derivative of thymidine.

AZT is an important anti-HIV drug serving as a reverse transcriptase inhibitor. It is reported to be a good substrate for TK1 with almost the same relative efficiency as Thymidine (8, 37). In cells treated with AZT, AZT is selectively phosphorylated by TK1, as TK2 has low capacity to phosphorylate AZT (6, 7). As the next step in the cascade - catalysed by thymidylate kinase - is very inefficient (8, 37), the product AZTMP (5'-monophosphate of AZT) is accumulated. AZT and AZTMP are stable metabolites - in blood serum or in cell extracts, thymidine phosphatase does not use AZT as a substrate (27).

Antibodies against AZT and AZTMP have been prepared and used in clinical settings to determine levels of these compounds in serum and extracts from tissues and cells (38, 39). However the use of AZT-specific and AZTMP-specific antibodies to determine TK1 activity has not been reported so far.

Furthermore, it is not state of the art to use 3'-derivatives of thymidine, in particular AZT, as a substrate for serum TK.

It has now surprisingly been found that 3'-derivaties of thymidine, in particular AZT, are selective, efficient and stable substrates for serum TK1.

In a second embodiment of this invention there is provided a method as disclosed before, wherein said substrate is a 3'-deoxy-thymidine derivative as shown in formula I, in which R is selected from but not limited to the group consisting of NH₂, NHCOCH₃, SC₂H₅, OC₂H₅, OBn, N₃, NO₂, OCOCH₃,OSO₂CH₃ and F.

Using a method for determining TK1 activity in a human or animal body fluid or cell or tissue sample as disclosed herein, 5'-phosphorylated 3'-thymidine derivatives are generated and the amount of said 5'-phosphorylated 3'-thymidine derivatives relates to the activity of TK1 in said sample. In the case that a sample comprising TK1 is reacted with AZT in the presence of a phosphate donor and a buffer system, AZTMP (5'-monophosphate of AZT) is generated and the amount of AZTMP formed relates to the activity of TK1 in the sample used. The amount of a 5'-phosphorylated 3'-thymidine derivative formed may be determined by any suitable method, in particular by an immunological method using an antibody with specificity to said 5'-phosphorylated 3'-thymidine derivative.

In a third embodiment of this invention there is provided a method as disclosed before wherein the 3'-derivative of thymidine is AZT and the 5'-phosphorylated 3'-derivative of thymidine is AZTMP.

In a forth embodiment, this invention relates to a method for determining thymidine kinase 1 activity in a human or animal body fluid or cell or tissue sample, which method comprises the steps of reacting said sample with a substrate for said thymidine kinase 1 which substrate is a 3'-derivative of thymidine in the presence of a phosphate donor and a buffer system and determining the amount of 5'-phosphorylated 3'-derivative of thymidine - said amount being related to said thymidine kinase 1 activity - by an immunological method comprising reacting the 5'-phosphorylated 3'-derivative of thymidine formed with at least one antibody capable of selectively reacting with the 5'-phosphorylated 3'-derivative of thymidine to form immunocomplexes.

In a variant of said embodiment of this invention, a method for determining thymidine kinase 1 activity in a human or animal body fluid or cell or tissue sample is claimed, which method comprises the steps of reacting said sample with a substrate for said thymidine kinase 1 which substrate is AZT in the presence of a phosphate donor and a buffer system and determining the amount of phosphorylated AZT (AZTMP) - said amount being proportional to said thymidine kinase 1 activity - by an immunological method comprising reacting AZTMP with at least one antibody capable of selectively reacting with AZTMP to form immunocomplexes.

According to this invention, immunocomplex refers to an antigen-antibody-complex. In particular, it refers to a complex comprising a 5'-phosphorylated 3'-derivative of thymidine and its specific antibody. It is particularly preferred that an immunocomplex comprises AZTMP and an antibody specific for AZTMP.

In context with the present invention, an immunological method refers to an analytical method based on immunochemistry, in particular it refers to an antigen-antibody reaction. According to this invention, immunological methods comprise but are not limited to immunoassays such as radio immunoassay (RIA), enzyme immunoassay (EIA), enzyme immunoassay combined with solid-phase technique (ELISA), immunofluorescence assay or chemiluminescence assay [The Immunoassay Handbook, Edited by David Wild, The Macmillam Press Ltd., 1994].

The immunoassay is expediently carried out firstly by exposing the sample to be investigated to an antibody selective for a 5'-phosphorylated 3'-derivative of thymidine, secondly by detecting and thirdly by quantifying the amount of antibody binding to the 5'-phosphorylated 3'-derivative of thymidine.

In a variant of this aspect of this invention, the immunoassay is carried out firstly by exposing the sample to be investigated to an antibody selective for AZTMP, secondly by detecting and thirdly by quantifying the amount of antibody binding to AZTMP.

In this type of assay, detection and quantification may be carried out directly or indirectly in a way known in the art using labelled antibodies specific for a 5'-phosphorylated 3'-derivative of thymidine, e.g. AZTMP, or using non-labelled antibodies specific for a 5'-phosphorylated 3'-derivative of thymidine, e.g. AZTMP, in combination with a secondary, labelled antibody specific for the primary antibody.

Thus, in a variant of this method, said antibody used to determine the amount of 5'-phosphorylated 3'-derivative of thymidine is labelled. In another variant of this method, said antibody is not labelled and detection and quantification is carried out using a labelled, secondary antibody with specificity for the primary unlabeled antibody. The handling and procedure of both variants of said immunological method is known to a person skilled in the art [The Immunoassay Handbook, Edited by David Wild, The Macmillam Press Ltd., 1994].

According to this invention, antibodies specific for a 5'-phosphorylated 3'-derivative of thymidine are immunoglobins with the ability to recognize and bind to a 5'-phosphorylated 3'-derivative of thymidine. Antibodies specific for a 5'-phosphorylated 3'-derivative of thymidine are preferably monoclonal or polyclonal antibodies. In particular, polyclonal antibodies specific for a 5'-phosphorylated 3'-derivative of thymidine are preferred.

According to this invention, antibodies specific for AZTMP are proteins with the ability to recognize and bind to AZTMP. Antibodies specific for AZTMP are preferably monoclonal or polyclonal antibodies. In particular, polyclonal antibodies specific for AZTMP are preferred. Polyclonal antibodies are known in the art or may be prepared according to processes known to the person skilled in the art (for example as described herein). It is preferred that said antibodies selectively react with AZTMP.

According to this invention, detection and quantification of the antigen-antibody complexes is made possible by using suitable labels known to a person skilled in the art - radioactive and non-radioactive, in particular a label selected from the group consisting of fluorescent dyes, enzymes, e.g. alkaline phosphatase or peroxidase, chemiluminescent compounds or radiolabels, e.g. ³H or ¹²⁵I.

In a further embodiment of this invention, there is provided a method for determining TK1 activity in a human or animal body fluid or cell or tissue sample, which method comprises the steps of reacting said sample with a substrate for said TK1 which substrate is a 3'-derivative of thymidine in the presence of a phosphate donor and a buffer system and determining the amount of 5'-phosphorylated 3'-derivative of thymidine by an immunological method using chemiluminescence.

In a variant of this invention, the immunological method is carried out with the aid of a suitable solid phase. According to this invention, suitable solid phase refers to glass and plastic particles. In particular it refers to magnetisable particles, tubes, wells and microtiter plates. In particular it refers to a customary commercial microtiter plate made of polystyrene customarily used for the ELISA technique.

In a further embodiment of this invention there is provided a method for determining TK1 activity in a human or animal body fluid or cell or tissue sample as disclosed herein, wherein the amount of said 5'-phosphorylated 3'-derivative of thymidine is determined by ELISA.

According to this invention, ELISA refers to any enzyme linked immunosorbent assay which is well known to a person skilled in the art.

In a variant, a method for determining thymidine kinase 1 activity in a human or animal body fluid or cell or tissue sample as disclosed herein is particularly preferred, wherein the amount of AZTMP is determined by ELISA.

In another variant of this immunological method, a solid phase is pre-treated with a secondary antibody and blocked by using a suitable blocking solution, e.g. gelatine, or a protein solution, saturating unspecific binding sites on the solid phase. Subsequently, the solid phase is incubated with an antibody solution which antibody has specificity for a 5'-phosphorylated 3'-derivative of thymidine, e.g. AZTMP.

When carrying out the method according to the invention with the aid of the ELISA technique, the antigen-antibody complexes are preferably detected and quantified via an enzyme-catalysed colour reaction using an enzyme selected from the group consisting of peroxidase or alkaline phosphatase. In particular, peroxidase in combination with TMB (3,3',5,5'-tetramethylbenzidine) is preferred.

According to this invention, immunological assays are carried out in a way that firstly the enzymatic TK assay is performed and secondly the product of said assay is transferred to a solid phase coated with capturing antibody to perform the detection reaction. In another variant, the enzymatic assay is performed directly on the solid phase coated with capturing antibodies.

In a further variant, detection and quantification of the antigen-antibody complexes is carried out by chemiluminescence and magnetic particles. In a given example an AZTMP derivative is coupled to an activated isoluminol yielding a product which product generates chemiluminescence and reacts with AZTMP antibody. This product is recognized by the AZTMP antibody as good as AZTMP itself and thus can be used as a detector molecule for a chemiluminescence system. It is known to a person skilled in the art how to couple antibodies to magnetic particles and how to generate chemiluminescence based on isoluminol. Such a TK assay - wherein magnetic particles are used for separation and chemiluminescence is used for detection - can easily be constructed instead of an enzymatic detection system in microtiter plates. Such a chemiluminescence assay is faster and more sensitive and covers a broader measuring range.

In a TK assay based on enzymatic detection, e.g. horseradish peroxidase, alkaline phosphatase or acetylcholinesterase, the presence of a reducing agent, e.g. DTE, DTT, or β-mercaptoethanol, is a requirement for an optimal TK activity reaction. Unfortunately, reducing agents can interfere with detection enzymes and thereby strongly reduce the detection signal and/or lead to non-reproducible results. A convenient way to overcome this problem is to add hydrogen peroxide to a final concentration of 0,05-0,25% during the immunodetection step. This will oxidise the interfering reducing agent and also improve robustness in the assay method as it stops the TK reaction.

In a further embodiment of this invention there is provided a method for determining TK1 activity in a human or animal body fluid or cell or tissue sample as disclosed herein, wherein said buffer system comprises at least Dithioerythritol (DTE), ATP, MgCl₂ and HEPES or Tris and provides a pH in the range from 5.0 to 9.0. In particular, a pH from 6.5 to 8.0 is preferred.

In another embodiment of the invention there is provided a method for determining TK1 activity in a human or animal body fluid or cell or tissue sample as disclosed herein, wherein said buffer system contains Uridine monophosphate (UMP) to protect phosphorylated substrates from being degraded by phosphatases which may be contained in the sample. Preferably the buffer system contains UMP in a concentration of at least 0,01 mM. In particular, a buffer system containing UMP in a concentration of at least 0,1 mM is preferred.

In another embodiment of this invention there is provided a method for determining TK1 activity in a human or animal body fluid or cell or tissue sample as disclosed herein, wherein the method is carried out with a substrate concentration is at least 0,4 µM. In particular, a substrate concentration of 2-500 µM is preferred. It is more particularly preferred that a substrate concentration of 20-200 µM is used in a method as disclosed herein. In a variant of this embodiment, an AZT concentration of more than 0,4 µM is preferred. In particular, a concentration of 2-500 µM AZT is preferred. It is more particularly preferred that a concentration of 20-200 µM AZT is used in said method.

In another embodiment of this invention there is provided a method for determining TK1 activity in a human or animal body fluid or cell or tissue sample as disclosed herein wherein said phosphate donor is present in a concentration of 0,1-10 mM. In particular, a method for determining TK1 activity as disclosed herein is preferred wherein said phosphate donor is present in a concentration of 0,5-5 mM.

In a further embodiment of this invention there is provided the use of a method for determining TK1 activity in a human or animal body fluid or cell or tissue sample as disclosed herein for diagnosing of diseases involving elevated levels of thymidine kinase 1 activity. It is preferred, that said method is used for diagnosis of cancer or tumours or for monitoring the progression of cancer or tumours.

According to this invention, tumour or neoplasm refers to tissue composed of cells that grow in an abnormal way. According to this invention, cancer refers to neoplasm or tumours that are malignant. In particular, cancer refers to haematological cancer, gastrointestinal cancer, breast cancer and prostate cancer.

In another embodiment of this invention there is provided a method for determining TK1 activity in a human or animal body fluid or cell or tissue sample as disclosed herein to diagnose cancer or tumours or to monitor the progression of cancer or tumours selected from the group consisting of haematological cancer, breast cancer, gastrointestinal cancer, or prostate cancer.

In a further embodiment of this invention there is provided a method for determining TK1 activity in a human or animal body fluid or cell or tissue sample as disclosed herein to identify a subgroup of patients which are at high risk of disease progression, e.g. Non-Hodgkin's lymphoma and chronic lymphatic leukaemia.

According to this invention, patients at high risk of disease progression in Non-Hodgkin's lymphoma and chronic lymphocytic leukaemia are those patients identified of having an increased TK1 activity level compared to healthy humans analysed by a method as disclosed herein and by using body fluid, cell or tissue sample.

A further embodiment of this invention refers to an in vitro method for diagnosing and/or therapeutic monitoring of diseases in human or animal characterized in having elevated levels of TK1 activity which in vitro method comprises the steps of obtaining a sample of human or animal body fluid or of a cell or tissue sample, assaying the sample to determine the TK1 activity according to a method as disclosed herein, and relating the amount of TK1 activity to the clinical status of the human or animal.

According to this invention, an in vitro method refers to a method for determination of a marker, whereby the determination is performed on human or animal material derived from human or animal body and tests are performed outside of the living organism wherefrom the material is derived.

According to this invention, diagnosing refers to determination of the nature of a disease or ailment.

According to this invention, therapeutic monitoring refers to monitoring the efficacy of treatment with anticancer agents. In particular it refers to monitoring the efficacy of treatment with anticancer agents by measuring the activity or expression of a biological marker such as TK1.

According to this invention, assaying refers to a quantitative method whereby the amount of a particular ingredient or substance is determined.

In a further embodiment of this invention there is provided a kit for the in vitro diagnosis and/or therapeutic monitoring of disease in human or animal characterized in having elevated levels of TK1 activity, which kit comprises a 3'-derivative of thymidine, a phosphate donor, a buffer, and at least one antibody capable of selectively reacting with the 5'-phosphorylated 3'-derivative of thymidine.

In another embodiment of this invention there is provided a kit for the in vitro diagnosis and/or therapeutic monitoring of disease in human or animal characterized in having elevated levels of TK1 activity, which kit comprises a AZT, a phosphate donor, a buffer, and at least one antibody capable of selectively reacting with AZTMP.

According to this invention, kit refers to a set or collection of tools, supplies or materials. In particular a kit comprises a 3'-derivative of thymidine, e.g. AZT, a buffer comprising a phosphate donor, and at least one antibody capable of selectively reacting with a 5'-phosphorylated 3'-derivative of thymidine, e.g. AZTMP. It is particularly preferred that kit refers to a set of materials comprising at least one TK calibrator, at least one TK control, at least one 3'-derivative of thymidine, e.g. AZT, at least one assay buffer comprising a phosphate donor, a stop reagent for TK activity, at least one primary antibody solution. Said primary antibody solution comprises antibodies against 5'-phosphorylated 3'-derivative of thymidine, e.g. AZTMP, and at least one tracer in a buffer comprising pyrimidine ring-labelled 3'-derivative of thymidine-5'-monophosphate, e.g. pyrimidine ring-labelled AZTMP, or at least one solid phase separator, e.g. tubes, microtiter plates or magnetic particles coated with secondary antibody, wash fluid, detection substrate or enhancement solution for an enzyme-, fluorescence-, or chemiluminescence-assay, at least one detection stop solution for an end point ELISA (e.g. an acid TMB stop solution), and sample diluent (a buffer for the dilution of patient sample).

In a further embodiment of this invention there is provided a kit as disclosed before additionally comprising UMP.

In another embodiment of this invention there is provided a kit as disclosed herein wherein said reagents are packed together in a container.

The invention will now further be described in detail with reference to figures 1-6.

### Description of figures

- Fig. 1: Structural formulas for the AZT derivatives AZXMP (Fig. 1 a); AZXMP-DSS (Fig. 1 b) and AZXMP-DSS-HRP (Fig. 1c). Structural formula for the activated isoluminol derivate ABEI-HS-ONS (Fig. 1d).
- Fig. 2: Anti-AZTMP antibody specificity. Dose response curve for the competitive assay, according to example 1, between free AZTMP (left curve, denoted AZTMP) or AZT (right curve, denoted AZT) and immobilised hapten, AZXMP (see Fig.1) illustrating the specificity of chicken anti-AZTMP antibodies. Y-axis: original absorbance values (B) are normalised against values obtained in the absence of competitor (B0) to obtain (B/B0).
- Fig. 3: AZTMP ELISA calibration curve. A competitive AZTMP end point ELISA assay was performed as described in example 4. The mean absorbance at 450 nm is plotted against standard concentrations of AZTMP. The 4PL curve fit formula is given with parameter estimates.
- Fig. 4: AZTMP formed as a function of TK1 activity. TK ELISA assays were performed
as described in example 6 using different concentrations of the substrate (AZT) in the TK reaction. The TK standard used was cytosolic TK1 standard from the Prolifigen^{®} TK REA kit. AZTMP standard was run in parallel, during the immuno-detection step, to allow the amount of AZTMP generated by the TK to be plotted against the TK activity. One graph is constructed for each AZT concentration (15, 20 or 30 µg/ml). The AZT concentrations refer to the concentration in the assay buffer.

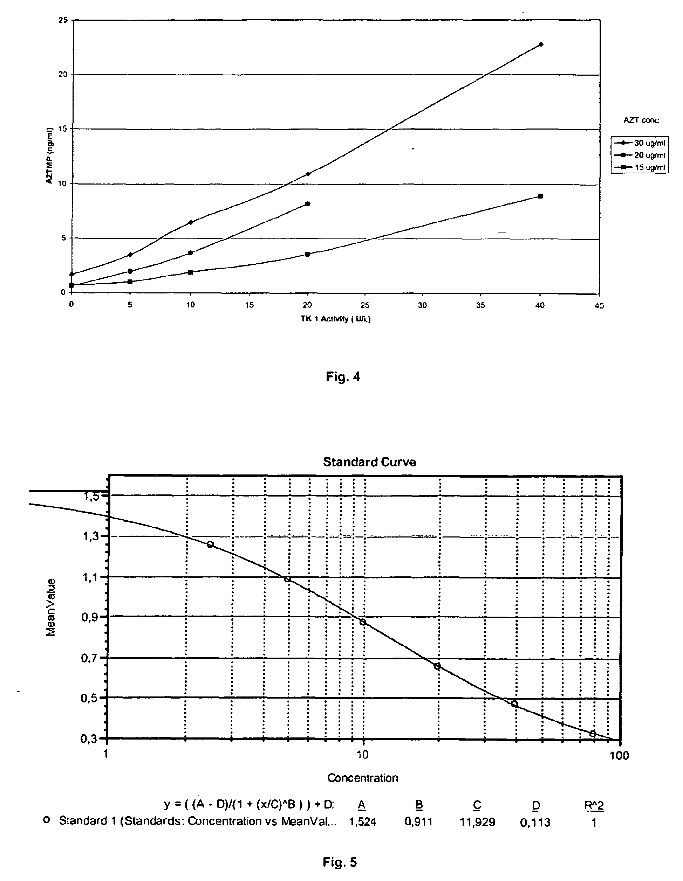

- Fig. 5: TK ELISA calibration curve. A competitive TK endpoint ELISA assay was performed as described in example 6. The mean absorbance at 450 nm (y-axis) is plotted against cytosolic TK1 activity in U/L using Prolifigen^{®} TK REA standard U/L values (x-axis). The 4PL curve fit formula is given with parameter estimates.

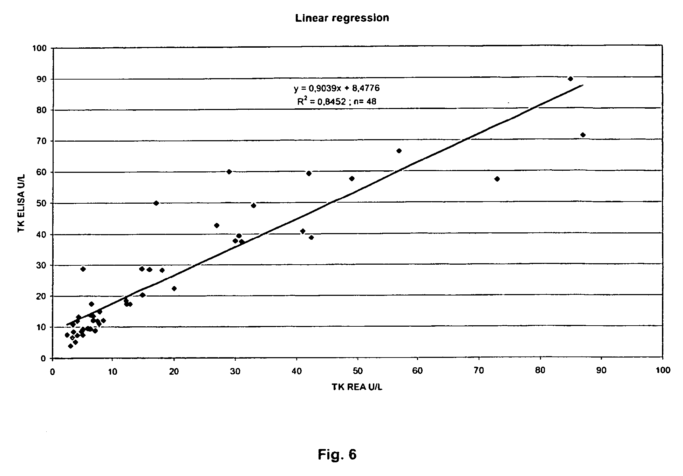

- Fig. 6: Linear regression analysis of TK ELISA vs. Prolifigen^{®} TK REA. Sera from 48 patients were tested in parallel with Prolifigen^{®} TK REA and the TK ELISA assay, described in example 6 and 7. The linear regression analysis yielded: TK ELISA = 0,9* (TK REA) + 8.5 (n = 48, r = 0,9)

### Abbreviations and notes

- ATP: Adenosine 5'-triphosphate
- AZT: 3'-Azido-3'-deoxythymidine
- AZTMP: AZT- 5'-monophosphate
- AZXMP: 3'-Azido-3'-deoxy-5'-monophosphate- ³N-1-(5-amino-pentyl) thymidine. Hapten molecule and ligand.
- KLH: Keyhole Limpet Hemocyanin. An adjuvant of the enhancement of the immunological reaction against the KLH-coupled ligand.
- EIA: Enzyme Immuno Assay diluent: 0,1 M phosphate pH 7.4 containing 0,15 M NaCl, 0.1 % Bovine serum albumin and 0,1 % Triton X 100
- PBST: 10 mM phosphate buffer pH 7.5 + 0.05 % Tween 20 (ELISA wash buffer)
- GA: Glutaraldehyd. Homobifunctionel coupling reagent.
- CNBr: Cyanogen Bromide
- BSA: Bovine Serum Albumin
- PB: Phosphate buffer. Concentration relates to [P]
- PBS: Phosphate Buffer Saline. Concentration relates to [P]
- ProCin 300: Preservative for controlling microorganisms in biological media
- HRP: Horse Radish Peroxidase. An enzyme for the transfer of 2H₂O₂ to 2H₂O + O₂.
This reaction oxidises TMB indicated by a change in colours.
- TMB: 3,3',5,5'-TetraMethylBenzidine. Is oxidized upon reduction of H₂O₂ by HRP. The change in colour upon oxidation is measured at wavelength 450 nm.
- DSS: Suberic acid bis(N-hydroxySuccinimide ester). A Homobifunctionel coupling reagent. Used for the coupling of AZXMP to HRP.
- RPC: Reversed Phase Chromatography. Liquid-flow chromatography using a polar mobile phase in conjunction with a non-polar stationary phase.
- PEG: Polyethyleneglycol
- Ph: Phenyl
- RT: Room Temperature
- PAGE: Poly acrylamide gel electrophoresis
- BIS: N,N'-Methylene-bis-acrylamide
- DTE: Dithioerythritol
- DTT: Dithiothreitol

### Example 1: Preparation and characterisation of antiserum against AZT-monophosphate (AZTMP)

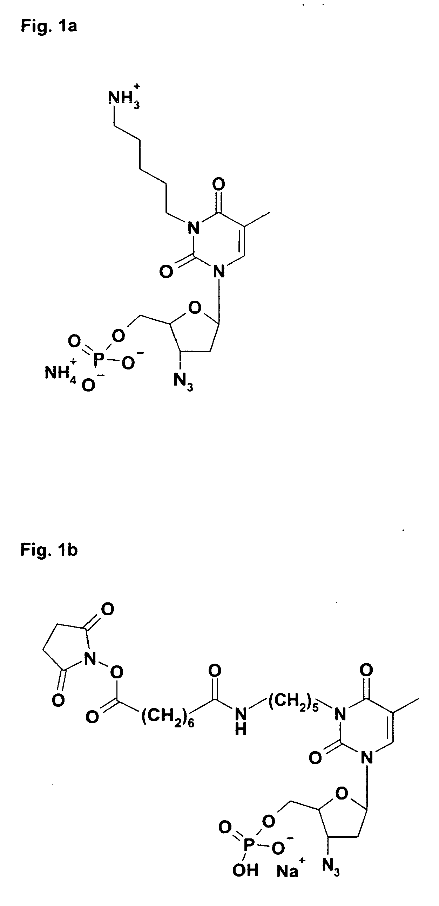

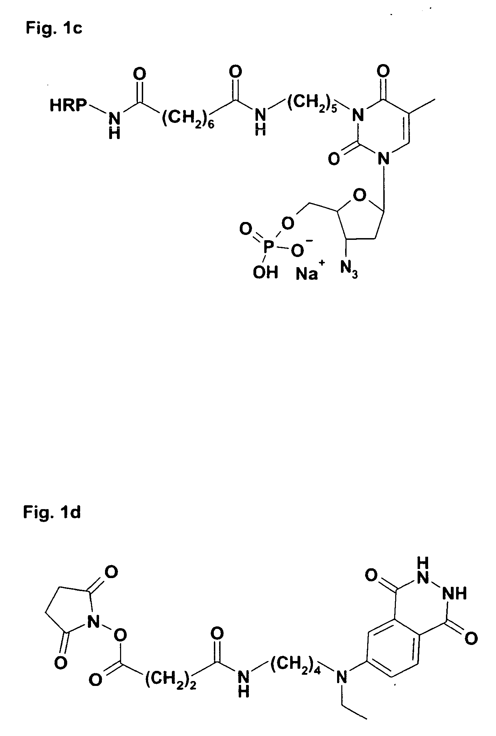

The preparation of polyclonal goat-antiserum and polyclonal IgY from hen eggs was performed according to a published method used in the preparation of rabbit antiserum (39). An AZTMP derivative, AZXMP (3'-Azido-3'-deoxy-5'-monophosphate- ³N-1-(5-amino-pentyl) thymidine) (39) (Fig.1a), was synthesised. The AZXMP was coupled to the carrier protein KLH (Keyhole Limpet Hemocyanin) enhancing the immunological reaction against the AZXMP hapten to form an antigenic conjugate KLH-AZXMP. Glutaraldehyd (GA) has been used to mediate coupling of KLH to AZXMP. The reaction was carried out at 6°C. A 2%-GA solution was added drop wise to AZXMP and KLH and incubated for 24 hours (h) under agitation. Subsequently the solution was dialysed three times against 0.1 M PBS (Phosphate Buffer Saline) pH 7,4 - resulting purified conjugate was frozen down to -80°C until using the material.

Hens and goats were immunized with 1,0 mg KLH-AZXMP. Goats were boosted in a regular 8-week cycles. Serum sample were taken each other week. Hens were boosted in a regular 4-week cycles. Eggs were collected continuously.

In order to stabilize the antibody preparation, hen-antibodies were precipitated by selective precipitation using PEG (Poly Etyhlen Glycol). Goat antibodies were affinity purified using AZXMP coupled to a CNBR (Cyanogen Bromide)-activated Sepharose Fast Flow column on an ÄKTA Explorer 100 system (Amersham Biosciences).

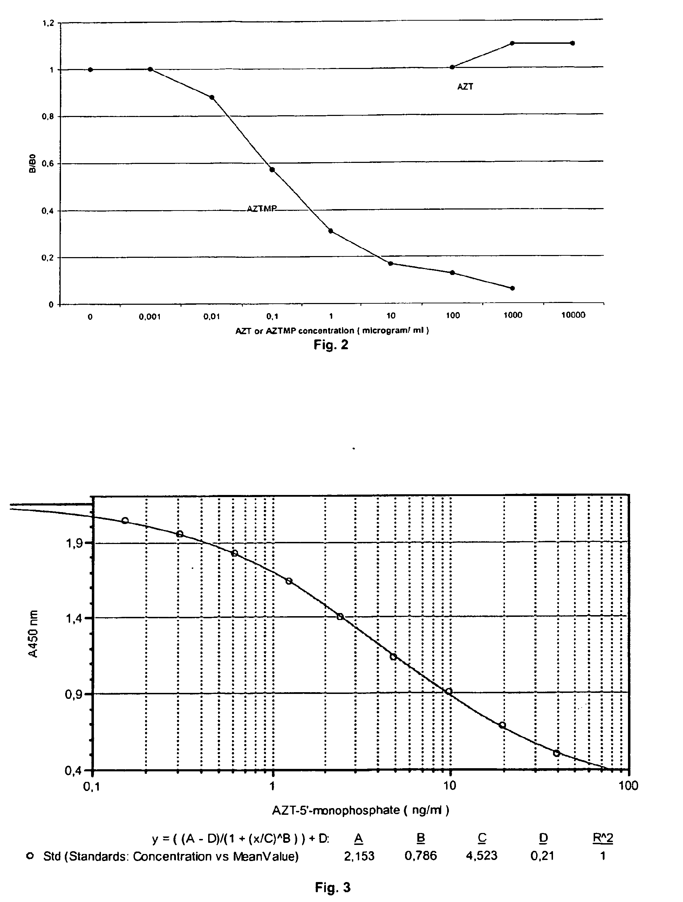

Antibody specificity and titre were evaluated using the competitive assay format described in example 4. To evaluate non-specific binding the antibody was substituted with diluent. Antibody specificity and titre was also evaluated using an assay whereby AZXMP hapten was coated to plates. AZXMP was conjugated to BSA using GA as linker and subsequently coated to the microtiter plates. In this assay, 100 µl of serially diluted antibody solutions were first incubated with AZXMP coated wells for 1h at RT. Wells of microtiter plates were washed three times with PBST (PBS plus 0,05% Tween 20). 100 µL of secondary antibody (diluted Horse Radish Peroxidase (HRP)-labelled anti-Goat IgG solution or anti-IgY antibody) was added and incubated for 30 min at room temperature. After 3 times washing with 250 µl PBST, bound antibody complexes were detected using TMB as substrate. The induced colour reaction was stopped after 5 min using 1M H₂SO₄ and detected at 450nm. Negative controls were performed by substituting the primary antibodies with diluent. Specificity of the antibodies was checked through the addition of increasingly amounts of AZTMP or AZT to the anti-AZTMP antibody solution. Non-5'-phosphorylated AZT was not able to compete out AZXMP for antibody binding (Fig.2). The specificity of the reaction was also shown by using serum samples taken from the immunised animals prior to immunisation with AZXMP-KLH. The results indicated that the obtained sera were specific for AZTMP and exhibit high avidity characteristics.

### EXAMPLE 2: Preparation of a stable tracer conjugate of AZXMP coupled to HRP

AZXMP was coupled to HRP using DSS (Suberic acid bis (N-hydroxySuccinimide ester)). This includes incorporation of an 8-carbon chain linker. Firstly, AZXMP was coupled through its primary amino group to one of the two active esters on the DSS molecule resulting in AZXMP-DSS (Fig 1b). In order to obtain an aequimolar coupling product an excess of 8 moles DSS per mole of AZXMP was used. The reaction was performed at 23°C in 1:1 diluent with equal parts of 100 mM sodium borate buffer pH 8.8 and pure acetonitrile. The coupling reaction was terminated after 30-60 minutes by adding 20 mM sodium dihydrogen phosphate, adjusting pH to 4,6 with 3 M HCl, and injecting the solution on the separation column yielding normally in 60-70% of AZXMP-DSS. Purifications were performed on a C2/C18 pepRPC reversed phase column (Amersham Biosciences). After adsorption under 20 mM sodium dihydrogen phosphate pH 4.6 the product was eluted with a 9:1-methanol/water-gradient. Fractions were pooled and the product was immediately frozen at -78°C and subsequently lyophilised in order to remove methanol used in the RPC step (Reversed Phase Chromatography). The product was reconstituted in 9:1 v/v H₂O/Acetonitril and immediately frozen and kept at -80°C until used. Concentration and purity of the final product was determined by RPC analysis using AZTMP as an internal standard.
AZXMP-DSS was conjugated to amino groups on HRP resulting in AZXMP-DSS-HRP (Fig. 1c). This was done in 50 mM borate buffer at pH 8.8 mixed with phosphate. For a standard reaction 15 moles of AZXMP-DSS were added per mole of HRP. The AZXMP-DSS-HRP conjugate was separated from low molecular weight material through gel filtration on a Superdex 200 HR 10/30 column (Amersham Biosciences) and 1 ml fractions collected. Appropriate fractions of AZXMP-DSS-HRP were pooled and diluted ten times in 10 mM PBS, 2,5% BSA (Bovine Serum Albumin), 0,1g/L K₃FeCN₆, 0,1 g/L bromophenol blue, 0,5% proclin 300 pH 7.4.

### Evaluation of enzymes and conjugation method

AZXMP was covalently coupled to AP (Alkaline phosphatase) using succinimidyl 4-(N-maleimidomethyl) cyclohexane-1-carboxylate (SMCC). 40 µl AZXMP (4.75 mg/ml) was mixed with 40µl of 0,2M Borate buffer pH 8,5 and 11 µl SATA (N-succinimidyl S-acetylthioacetate, Sigma, 15 mg/ml in acetonitrile). After 75 minutes at room temperature deacetylation was started by adding 45,5 µl 0,5M hydroxylamine, 25 mM EDTA, 50 mM phosphate buffer pH 7.5 and 45,5 µl 0,1 M phosphate buffer pH 6.65. After additional 75 min 13,65 µl 0,3 M HCl was added to adjust pH of 6.4. The reaction mixture was injected to a pepRPC HR 5/5 column (Pharmacia). Separation was performed with ÄKTA Explorer system 100 and a linear gradient of 0-100 % 9:1 v/v methanol/H₂O in 15 column volumes. Fractions were collected and the thiolated AZXMP recovered. The thiol content of the product was measured colourimetrically after reaction with 5-5'-dithio-bis-nitrobenzoate (DTNB) (44). After chromatography the methanol was evaporated and the thiol content was measured again.
Conjugation of the thiolated AZXMP with the enzyme EZ-link^{™} maleimide activated Alkaline Phosphatase was obtained by mixing a 10 molar excess to the enzyme (0,5 mg) in 0,1 M phosphate buffer, 2.5 mM EDTA pH 6,9. After over night reaction at 4°C the conjugate was purified by gelfiltration on a superdex 200 HR 10/30 column (Amersham Bioscience) and stored at -20°C until use.
AZXMP was also conjugated to HRP using pre-activated HRP from Pierce (Rockford, USA). 36,5 µl AZXMP (4.75 mg/ml) was diluted with 0,1 M PBS pH 7,2 to give 500µl. This solution was used to reconstitute 1 mg EZ-link^{™} Plus activated peroxidase. 10 µl 0,255 M ascorbic acid was immediately added for reduction of the schiff base. After 60 minutes reaction at room temperature 20µl 0,6 M Tris pH 9.0 was added to terminate the reaction. After 20 minutes 500 µl of the reaction mixture was loaded on a Superdex 200 HR 10/30 gel filtration column. Separation was performed using an ÄKTA Explorer 100 chromatography system complemented with a frac 900 fraction collector. Fractions containing monomeric HRP were collected and diluted 10 times in 10 mM PBS, 2,5% BSA, 0,1g/L K₃FeCN₆, 0,1 g/L bromophenol blue; 0,5% proclin 300 pH 7.4.

AZTMP assays were then performed as described in example IV with antibodies described in example I and the AZXMP tracer conjugates compared. For the assay with AP p-nitrophenylphosphate (pNPP) was used as the substrate.

**Table 1. Comparison of AZXMP tracer conjugates.**

| Tracer conjugate | detection time | Conc. of added enzyme | signal forB0 |
|---|---|---|---|
| AZXMP-AP | 30 min | 600 ng/ml | 0,27 AU |
| AZXMP-HRP (aldehyde activated) | 20 min | 2 000 ng/ml | 0,5 AU |
| AZXMP-HRP (using DSS as a linker) | 5 min | 150 ng/ml | 2.5 AU |

| | | | |
|---|---|---|---|
| (BO is the absorbance signal for the zero standard giving the maximum value in competitive assay.) | | | |

The results show that the AZXMP-HRP tracer conjugated with the DSS linker method is superior in sensitivity. The use of conjugated HRP is also preferred in comparison with AChE (Acetylcholinesterase) since HRP allows substantially shorter detection times (10 minutes compared to more than 30 minutes for AChE).

### EXAMPLE 3: Serum TK measurement with tritium labelled AZT and correlation to the TK-REA.

In order to determine if serum TK can use AZT as substrate, an experiment was performed with 16 clinical patient serum samples (20 µl) mixed with 2 µL ³H-AZT (Ci/mmol Moraveck inc, USA) and 20 µL reaction mixture containing 10 mM Tris-HCl (pH 7.6), 5 mM ATP, 5 mM MgCl₂, 3.5 mM NaF, 2 mM DTT. The assay was performed 4 h at 37°C and the amount of AZTMP formed was determined by DE 51 ion exchange paper method as described (9). Previous experiments had established that this amount of serum gave a linear reaction during the time period tested. The results as well as the results from the TK-REA assay are shown in Table 6.

**Table 6. Comparison of substrates for serum TK activity**

| Sample | ³H-AZT (CPM) | Prolifigen® TK-REA (U/I) |
|---|---|---|
| 1 | 726 | 2.8 |
| 2 | 450 | 3.1 |
| 3 | 916 | 2.5 |
| 4 | 2188 | 2.9 |
| 5 | 1714 | 13.8 |
| 6 | 1664 | 12.3 |
| 7 | 1804 | 12.6 |
| 8 | 2147 | 16.2 |
| 9 | 9178 | 54.1 |
| 10 | 11447 | 54.6 |
| 11 | 4107 | 34.2 |
| 12 | 6458 | 58.1 |
| 13 | 12937 | 141 |
| 14 | 13270 | 692 |
| 15 | 294322 | 1620 |
| 16 | 5239 | 345 |

The results show an overall good correlation between the activity levels measured with AZT and ¹²⁵IdUrd in the Prolofigen® TK-REA - the correlation coefficient for a linear regression was 0,9 with TK REA values below 200 U/L - apart from sample 4, 12, 14 and 16, whereby sample 4 resulted in a higher activity with AZT than with IdUrd and samples 12, 14 and 16 resulted in lower activities than those found with the TK-REA. The reason(s) for these discrepancies are not known but the conditions in the two types of assays are quite different. This fact may explain some of the discrepancies but the presence of interfering enzymes such TK2, thymidine phosphorylase or viral deoxynucleoside kinases may also be responsible. We concluded from this experiment that serum TK can efficiently use AZT as substrate.

### EXAMPLE 4: AZTMP ASSAY

### Coating of microtiter plates.

Secondary antibody (either rabbit anti-goat or rabbit anti-chicken or goat anti-chicken antibody) was diluted in 0,1 M hydrogen carbonate buffer pH 8.5 to 5 mg/L. Nunc C8 microtitre strip well plates were incubated with 100 µl of antibody solution over night (17-24 h) at 2-8°C. Plates were washed 3 times with 250 µl PBS. Finally 250 µl EIA (Enzyme Immuno Assay diluent) buffer was added for blocking. Plates were stored at 2-8 °C. Before use, the plates were washed 3 times with 250 µl PBST.

### Assay

The assay was performed in micro titre plates coated with either an anti IgG antibody or an anti IgY antibody. The total reaction volume was 150 µl and each component (HRP-DSS-AZXMP tracer, anti-AZTMP antibody and AZTMP standard) was added in a volume of 50 µl. A 1:1000 dilution of the tracer was used corresponding to 70 ng HRP/ml. The anti-AZTMP antibody was diluted to 200 ng/ml. The immunoreactions were performed at RT under mild agitation (300-400 rpm) for 2h. After wash 3 times with 250 µl PBST 100 µl TMB (5,5'-TetraMethylBenzidine). The enzymatic reaction was stopped after ten to fifteen minutes by addition of 100 µl TMB stop (Bio Fx, USA) and the resulting absorbance was read at 450 nm.

Figure 3 show a typical standard curve for the AZTMP assay.

### EXAMPLE 5: Interference from reducing agents in the AZTMP assay based on enzymatic detection

Acetylcholinesterase (AChE) tracer and rabbit anti-AZTMP (rabbit 1290, bleeding # 7) was bought from SPI-Bio, Saclay France. The AZTMP assay (39) was performed with the modification that micro titre plates were coated with 2 µg/well goat anti-rabbit IgG. AZTMP assays were performed in which AZTMP standards were diluted in TK assay buffer containing 86 mM HEPES, 31 mM NaOH, 7,8 mM MgCl₂^{*}6H₂O,17 mM KCI, 71 mM Mannitol, 3,9 mM ATP and 6 mM DTE. The results were compared to those were AZTMP standard was diluted in EIA buffer. It became obvious that the DTE interfered with the AZTMP assay by lowering the detection signal.

Zero standard signals for AZTMP Assay. Absorbance at 405 nm

**Table 2. Interference from DTE in the AZTMP assay.**

| EIA Buffer | TK Substrate buffer (DTE excluded) | TK substrate buffer (DTE included) |
|---|---|---|
| 0,42 | 0,36 | 0,2 |

DTE (or a similar) reducing substance that is essential for the TK reaction had to be eliminated prior to the AZTMP assay to give reproducible results. An experiment was performed were the Ellman reagent (45) was used as indicator to titrate the amount of sodium hypochloride needed to oxidise the DTE used in the TK reaction. After reduction of the DTE with sodium hypochloride, the results in the AZTMP assay were comparable with those results where the EIA buffer was used to dilute the AZTMP standard. The same type of experiment has been performed with AZTMP as described in example 4 based on reagents in example 1 and 2 with the same results - namely that the DTE reduces the absorbance signal.
In the case that the AZTMP assay as described in example 4 was modified in the way such that the primary antibody was allowed to bind to the secondary antibody first followed by a wash step and finally AZXMP tracer conjugate was added in the sample containing AZTMP, then the interference did not occur.
In another experiment the solid phase antibody, primary antibody against AZTMP and the enzyme labelled AZXMP were exposed to DTE treatment. After one hour DTE was removed by dialysis and AZTMP assays were performed with or without components exposed separately to DTE. These experiments showed that in contrast to HRP or acethylcholine esterase the antibodies still worked effectively after exposure to DTE.

### EXAMPLE 6: Correlation between TK1 in the sample and the amount of AZTMP detected. The use of hydrogen peroxide for enzymatic detection.

TK1 standard: 10⁷ HeLa cells/ml were harvested by centrifugation from the growth medium after trypsination. The pelleted cells were separated from the supernatant and resuspended in PBS. After a second centrifugation the cells were resuspended in lysis buffer containing 50 mM HEPES, 5 mM DTE, 2 mM ATP and 2 mM MgCl₂*6H₂0. The cell suspension was subjected to five freeze-thawing cycles at - 70 °C and 25°C, respectively. The ruptured cells were centrifuged at 48 000g for 1 h at +2°C to pellet cellular debris. The supernatant containing TK and soluble protein was decanted. To check that no TK2 material was present, electrophoresis was performed followed by a Prolifigen® TK REA Assay (AB Sangtec Medical, Bromma Sweden). A sample comprising 100 µl TK cytosol + 40µl 50% glycerol + 5 µl 1% Bromophenolblue was placed on a 5% PAGEs with 0.25% BIS (N,N'-Methylene-bis-acrylamide) and electrophoresis was performed in a buffer containing 190 mM Glycin, 200 mM ATP, 25 mM Tris, 1 mM MgCl₂, 3 mM β-Mercaptoetanol pH 8.6 for 3 hours and 100 V. Electrophoretic results were sliced in 45 pieces of 1,5 mm thickness and mixed with TK REA assay buffer whereby the enzymes were extracted. The TK REA assay was run and the radioactivity for each slice calculated and plotted against the relative mobility. The material showing no presence of TK2 was diluted in foetal calf serum and lyophilised.

### Substrate buffers

TK assay buffer: 86 mM HEPES, 31 mM NaOH, 7,8 mM MgCl₂*6H₂O, 17mM KCl, 71 mM Mannitol, 3,9 mM ATP and 6 mM DTE.

Substrate buffers for the TK reaction were prepared by adding AZT to the assay buffer in the following concentrations: 1; 5; 15; 20; 25; 30 µg/ml.

### TK reaction

A 80 U/I TK1 standard preparation (in heat inactivated fetal calf serum) was diluted in 9 g/I NaCl to give further standard levels of 40, 20, 10, 5 and 2,5 U/L, respectively. Sodium chloride was also used as zero standard level. TK reactions were performed by adding 50 µl of substrate buffer and with 50 µl of TK1 standard in non-coated micro wells. The plate was incubated for 60 minutes at 37 °C.

### Stop reaction

After 60 minutes incubation, 50 µl of 0.25% H₂O₂ was added to each well to react for at least 5 minutes at RT resulting in oxidization of DTE and subsequently effectively terminating TK reaction - since the TK enzyme is sensitive to an oxidative environment.

### Detection of generated AZTMP

To detect and quantify generated AZTMP (based on TK1 activity), 50 µl of the reaction mixture was transferred to a plate coated with secondary antibody and mixed with 50 µl anti-AZTMP antibody (200 ng/ml diluted in EIA buffer) and with 50 µl AZXMP-DSS-HRP tracer conjugate, 300 ng HRP/ml, diluted in 10 mM PBS, 2,5% BSA, 0,1g/L K₃FeCN₆, 0,1 g/L bromophenol blue, 0,5% proclin 300 pH7,4. The plate was incubated for 2h at RT under mild agitation then washed 3 times with 250 µl PBST. Finally 100 µl of TMB reagent was added and after 10 minutes 100 µl TMB stop (BioFx. USA). The absorbance was read at 450 nm using a standard plate reader.

After TK reaction, generated AZTMP (from the TK1 standard) was plotted as a function of the TK concentration. The amount of AZTMP generated was directly proportional to the amount of TK1 present in the sample and the turn over rate was dependent on the AZT concentration (Fig 4.).

A typical standard curve for the TK ELISA is shown in Figure 5 - using as standard material the standard of the TK REA kit. In this example a substrate concentration of 50 µg/ml AZT was used in the assay buffer.

Using the assay described above, the concentration range for hydrogen peroxide was investigated.
The assay was found to be surprisingly stable in a broad concentration range (final concentration during the immuno-reaction step) from 0.015% to 0.25% (and even higher concentrations) hydrogen peroxide. Excess hydrogen peroxide increased the signal. Stabilised hydrogen peroxide - an environmentally friendly reagent - was stable in this concentration range and thus suitable as a component in a kit. We conclude that the use of low concentrations of hydrogen peroxide is an excellent way to stop the TK reaction and to facilitate the AZTMP detection using enzymatic detection for a TK ELISA.

### EXAMPLE 7: Evaluating the TK Assay with patient sera

TK standards were prepared as described in example 6. TK standard (80 U/L in fetal calf serum) was diluted with 9 g/l NaCl to 40, 20, 10, 5, and 2,5 U/L, respectively. 9 g/l NaCl was used as a negative control. Sera from 48 patients - tested with TK REA - were assayed in parallel with TK REA and in the described assay called TK ELISA.

50 µl of sera or standard was added to 50 µl of a substrate buffer containing 172 mM HEPES, 62 mM NaOH, 15,6 mM MgCl₂*6H₂O, 34 mM KCI, 142 mM Mannitol, 7,8 mM ATP, 12 mM DTE and 50 µg/ml AZT. After 60 minutes reaction at 37°C in uncoated micro titre wells, the reaction was stopped with 0,25% hydrogen peroxide. After five minutes 50 µl of the solution was transferred to a microtiter plate coated with rabbit anti goat antibodies together with 50 µl of AZXMP-DSS-HRP tracer and 50 µl polyclonal goat anti-AZTMP. After 2 hours incubation with mild agitation the plate was washed 3 times with PBST. 100 µl TMB was added and the reaction was stopped after ten minutes by adding 100 µl TMB stop. The tracer diluent was supplemented with 100 mg/L bovine IgG, 100 mg/L rabbit IgG and 0,25 mg/L goat IgG since this tested formulation could suppress a panel of human sera containing heterophilic antibodies. The results gave a significant correlation between the methods (Fig 6). The TK ELISA can be used for serum measurement.

Next patient sera were screened with TK ELISA and TK REA. Five patient sera were measured significantly higher with TK ELISA than with the TK REA

**Table 3 Patient samples showing significant discrepancy between the TK ELISA method and Prolofigen® TK REA method.**

| Sample | TK REA (U/L) | TK ELISA (U/L) | TK ELISA (No substrate) |
|---|---|---|---|
| 1 | 29 | 60 | 11,8 |
| 2 | 6 | More than 80 | 1,5 |
| 3 | 17 | 50 | Not detectable |
| 4 | 5,1 | 29 | Not detectable |
| 5 | 7,9 | 14,9 | Not detectable |

To investigate whether values generated with the TK ELISA were generated from TK1 activity or from interference in the assay, samples 1-5 were retested. The assay was run with buffer instead of AZT in the TK reaction step. After that hydrogen peroxide solution was added the concentrated AZT was spiked back into the sample prior to the immuno-detection to give the same conditions as when the AZT was included in the TK reaction step.
The results (TK ELISA (No substrate)) are shown in Table 3. The detection limit for the ELISA is approximately 1 U/L. Only sample 1 gave a false positive signal of 11,8 U/L but a true positive signal of 48,2 U/I.

Using a cut off value of 10 U/L, all five samples were tested positive in the TK ELISA. Only one out of five samples were tested positive in the Prolofigen® TK REA. Therefore, the TK ELISA assay clearly demonstrated its potential for more valid results.

### EXAMPLE 8: Inclusion of Uridine monophosphate (UMP) to inhibit phosphatase activity.

A potential problem for the analysis of a kinase activity in patient sera is the presence of phosphatase activity in the same sera to drive the reverse reaction (dephosphorylation). One way to inhibit the phosphatase is to include UMP in the substrate buffer to protect AZTMP from being dephosphorylated to AZT.

Substrate buffers containing 172 mM HEPES, 62 mM NaOH, 62 mM MgCl₂*6H₂O, 34 mM KCI, 142 mM Mannitol, 7,8 mM ATP, 12 mM DTE, and 25 µg/ml AZT were prepared - with variable concentrations UMP, i.e. 0, 0.1, 1 or 5 mM UMP.
A TK1 standard preparation 80 U/L (in heat inactivated fetal calf serum) was diluted in 9 g/L deionized-water, also used as zero standard level, to give further standard levels of 40, 20, 10, 5 and 2,5 U/L, respectively. Four human sera were also assayed. Two sera were from healthy blood donors and two sera from patients monitored with TK REA.

Next the TK reactions were performed by adding 50 µl of substrate buffer together with 50 µl of TK1 standard/or patient serum sample in non-coated microtitre wells.
The plate was incubated for 60 minutes at 37°C. Afterwards 50 µl of 0.25 % hydrogen peroxide was added to each well and allowed to react for at least 5 minutes at RT.

For the detection 50 µl were transferred to a plate which plate was coated with 1 µg/well rabbit anti chicken IgY (Sigma, MO, USA). 50 µl of chicken anti-AZTMP (diluted 1:1000 in EIA buffer) and 50 µl of AZXMP-DSS-HRP tracer (diluted 1:1000 in 10 mM PBS, 2,5 % BSA, 0,1g/L K₃FeCN₆, 0,1 g/L bromophenol blue, 0,5% proclin 300 pH7,4) were added. The immuno reaction was performed under mild agitation and was stopped after 120 minutes by washing the plate 3 times with 250 µl PBST followed by addition of 150 µl TMB (one component) solution. The TMB reaction was stopped with 100 µl acid TMB stop and the absorbance read at 450 nm.

**Table 4. The use of Uridine Mono Phosphate (UMP) to inhibit phosphatases.**

| **Absorbance for standards at 450 nm in milli absorbance units** | | | | |
|---|---|---|---|---|
| | | | | |
| **Conc. TK U/L** | **Conc. UMP mM** | | | |
| | **0** | **0,1** | **1** | **5** |
| **80** | 130 | 132 | 138 | 135 |
| **40** | 190 | 194 | 190 | 188 |
| **20** | 292 | 297 | 295 | 264 |
| **10** | 426 | 451 | 432 | 422 |
| **5,0** | 638 | 640 | 640 | 620 |
| **2,50** | 774 | 810 | 895 | 839 |
| **1,25** | 949 | 933 | 1010 | 920 |
| **0,00** | 1250 | 1300 | 1230 | 1065 |

| **Samples concentration U/L** | | | | |
|---|---|---|---|---|
| | | | | |
| **Sample** | **Conc. UMP mM** | | | |
| **No** | **0** | **0,1** | **1** | **5** |
| **1** | 1,36 | 1,09 | 2,69 | 2,11 |
| **2** | 8,31 | 8,39 | 7,82 | 8,22 |
| **3** | 3,21 | 3,34 | 4,46 | 4,90 |
| **4** | 3,35 | 3,09 | 3,58 | 3,98 |

The standard curve was not affected by the addition of UMP however two of the tested sera gave significantly higher TK values after addition of 1 mM UMP. We conclude that inclusion of 1 mM UMP in the substrate buffer, for the TK reaction, might give an extra protection against degradation.

### EXAMPLE 9: Differences between cytosolic TK and serum TK observed with antibodies.

A polyclonal anti-TK antibody was evaluated for the measurement of serum TK. The configuration was to use the anti.-TK1 antibody as a solid phase capture antibody and then run the enzymatic reaction of the immobilized enzyme. The preparation of the antibody is described elsewhere (32). The antibody binds to an epitope located on C-terminal part of the TK1. Therefore, TK activity was not blocked by binding of the antibody to TK.
Immunosorbent tubes (Greiner,Germany) were coated with 2 µg antibody/tube in a coating volume of 200 µl. Negative control tubes were made at the same time coated with BSA instead of antibody. A test procedure was then performed as follows. 50 µl of standard or sample containing TK1 were incubated together with 150 µl BSA/PB diluent in an antibody coated tube for 2 hours at 37°C. Tubes were washed 3 times with 2 ml 9 g/L NaCl and subsequently 200 µl TK REA tracer (containing 5-lodo-(¹²⁵I)-deoxyuridine as the radioactive substrate) were added and incubated for two hours at 37°C. After the incubation, 300 µl water were added to result in a total volume of 500 µl. The separation and wash procedure for the TK REA assay was followed. Briefly the generated 5-lodo-(I-125-)-deoxyuridine monophosphate was adsorbed to a separator tablet, washed and the radioactivity was measured in a gamma counter.

Cytosolic extracts from human beast tumour tissue and sera from individual cancer patients diluted 1:10 w/v in fetal calf serum were assayed after the procedure as described. A TK1 standard level of approximately 360 U/L was also produced as described in example 6 and assayed together with TK REA standard of 0, 8 and 80 U/L, respectively. All samples were run in duplicates

**Table 5. Evaluation of a polyclonal anti-TK1 antibody as a capture antibody.**

| Sample | PAbTK1 [Bound cpm] | B/T [%] | TK REA [U/L] |
|---|---|---|---|
| Std 0 U/L | 281 | 0,67 | 0 |
| Std 8 U/L | 360 | 0,87 | 8 |
| Std 80 U/L | 924 | 2,22 | 80 |
| Std high | 2856 | 6,86 | 360 |
| Serum 1 | 249 | 0,6 | 5,7 |
| Serum2 | 517 | 1,24 | 51,7 |
| Serum3 | 1413 | 3,39 | 1300 |
| Serum4 | 924 | 2,22 | 1100 |
| Cytosol 1 | 1990 | 4,78 | 300 |
| Cytosol 2 | 1591 | 3,82 | 250 |

The BSA coated tubes used as negative controls showed no binding for any of the samples. The TK1 standard material obtained from cytosols gave a good correlation to TK REA (TK REA values correlated against pAbTK1 bound cpm). The cytosol samples 1-2 showed an expected "pABTK1" activity in the same level as the standards in relation to the TK REA activity whereas the sera showed lower activity than expected. The activity measured after the capture was generally rather low in particular for the serum samples. When the procedure as described above was used, i.e. use of a TK1 antibody for capturing, the activity obtained for the serum sample was too low for a sensitive and reliable assay. More importantly, different results were obtained from serum TK1 and cytosolic TK1. This illustrated the problem of using antibodies for the quantification of TK in serum. The results were not surprisingly since TK is reported to be present in large complexes in serum.

### EXAMPLE 10:Assay format excluding sample transfer

The TK assay, described in previous examples, was based on the TK reaction in an uncoated micro well. This was done to get a better control since DTE present in the TK assay buffer might interfere negatively by sulfhydryl exchange. Since the coated antibody could withstand the condition during the TK reaction, a simpler "direct" assay was performed. 50 µl of patient sample or standard was added directly to the micro wells coated with secondary rabbit anti chicken antibody together with 50 µl substrate buffer. The wells were coated as described in example 4 and assay buffer was prepared according to example 4. The TK reaction was allowed to proceed for 60 minutes at 37 °C. Then the micro titre plate was placed at room temperature (23 °C) and 25 µl 0.50% (w/w) H₂O₂ were added to terminate the TK substrate turn over. After 5 minutes reaction time for the hydrogen peroxide with a mild agitation, 50 µl of primary antibody solution (IgY anti AZTMP) and 50 µl tracer conjugate were added sequentially and the plate was incubated for additional 2 hours at RT. The plate was washed 3 times with 250 µl PBST. 100 µl TMB substrate was added and after fifteen minutes the colour development was terminated by addition of 100 µl TMB stop solution and the absorbance was read at 450 nm. Compositions and dilutions for the tracer and primary antibody solution as previously described in example 6 were used - with the modification that for the primary antibody solution bovine IgG to a final concentration of 25 mg/L was added as protection against heterophilic antibodies that could be present in human sera.

### EXAMPLE 11: Assay based on chemiluminescence and magnetic particles.

AZXMP was conjugated to Isoluminol using the activated derivate of Isoluminol named ABEI-HS-ONS (Fig. 1d) provided by Byk-Sangtec Diagnostica GmbH (Dietzenbach, Germany). Conjugation was accomplished by adding 25 µl of 0,64 mg/ml AZXMP in 0,1 M Borate buffer pH 8,8 to 25 µl ABEI-HS-ONS dissolved in acetonitrile. The mole ratio was 1,6 moles of AZXMP per mole ABEI-HS-ONS. The reaction mixture was thoroughly mixed and then allowed to stand for 60 minutes at 22°C. Prior to isolation of the conjugation product, using RPC (reversed phase chromatography), 500 µl of chromatographic eluent (A = 20 mM KH₂PO₄/HCl pH 2.0) was added and 200 µl of the sample injected on a pepRPC HR 5/5 column (Amersham Bioscience). A linear gradient of 0-100 % of B (B = 10:1 methanol /water) in 15 column volumes was generated over the column. 1 ml fractions were collected and the peak corresponding to the AZXMP-HS-ABEI fraction collected.
2 ml 13 mM PBS pH 7.5 were added resulting in a pH of 6.5. The preparation was frozen and kept at - 78 °C until used. An aliquot was diluted 10201 times in PBS and the chemiluminescence generated by the compound measured. Measurement was performed on a S300 Luminometer system (Berthold, Germany) using starter reagents from Byk-Sangtec Diagnostica GmbH (Dietzenbach, Germany). 100 µl of the diluted sample generated 107000 relative luminescence units. A diluent for the tracer conjugate containing 50 mM phosphate buffer, 0.5 % (v/v) BSA , 25 mg/L bovine IgG, 0,09 % sodium azide, 0.02 % Tween 20, pH 7,5 was prepared. After thawing the AZXMP-HS-ABEI 1 ml was diluted with 100 ml diluent to give a working tracer solution # GM-02-072 of approximately 0,12 µM. Tracer # GM-02-272 was diluted serially in two fold dilutions in its tracer diluent. These dilutions were then run as samples in an AZTMP ELISA and the results calculated from the AZTMP standard curve.

**Table 7. Functionality test of an isoluminol conjugated AZTMP derivate (AZXMP-HS-ABEI).**

| Dilution | obtained AZTMP conc ng/ml |
|---|---|
| Undiluted | 50,4 (out of range) |
| 1:2 | 22,4 |
| 1:4 | 10,0 |
| 1:8 | 5,0 |
| 1:16 | 2,2 |
| 1:32 | 1,0 |
| 1:64 | 0,44 |
| 1:128 | 0,21 |

The AZTMP-HS-ABEI is recognised equally well as AZTMP by the assay. Thus it can be used as an effective tracer molecule in a chemiluminescence assay. Secondary antibodies can easily be coated to magnetic particles. AZTMP and TK assays using the principles in the previously described examples can be developed.

### EXAMPLE 12: Clinical determination of TK in cvtosol/tissue extract

The TK1 concentration was determined in cytosol/tissue extracts from patients with non-metastatic breast cancer. TK analysis might refine the individual prognosis and treatment of breast cancer patients as TK is an independent prognostic factor in breast cancer patients receiving adjuvant chemotherapy. TK activity was found to increase dramatically in breast cancer relative to normal breast tissue. High TK levels are an important risk factor in node-negative breast cancer patients and seem to be associated with a beneficial effect of adjuvant treatment.
Tumour samples were obtained preoperatively and stored on dry ice. The tissue was pulverized - the frozen powder was dissolved in a buffer (10 mM Tris-HCl, 1.5 mM EDTA, 5 mM Na₂MoO₄ and 1 mM monothioglycerol, pH 7.4). The homogenate was centrifuged at 105000xg. In order to stabilize the TK activity and improve the linearity of dilutions, cytosols were diluted 1:20 in heat-inactivated normal calf serum containing 90 mM HEPES, 18 mM KCI, 6 mM Dithiotreitol, 8 mM MgCl₂ and 4 mM ATP. The TK assay was performed as described in example 6. The TK levels were expressed in mU/mg protein in the cytosol.

### Commercial utility

Serum TK levels have a proven role in cancer diagnostics, provide prognostic information for relapse free survival and over all survival and in some cases aid the choice of therapy (17-24). A recent example is the identification of a subgroup at high risk of disease progression in chronic lymphocytic leukaemia because of high serum TK levels (18). In a series of studies from France it was demonstrated that cytosolic TK1 levels was the best predictive marker for progression of disease in a large number of breast cancer patients (20-24).

The present method according to the invention allows the determination of TK1 activity in a sample in an advantageous manner. This type of method is fast, simple and very sensitive due to the high efficiency of TK1 to phosphorylate these selective substrates. The reagents and reaction products are stable, non-radioactive and non-hazardous and can be detected in large capacity assay formats.
The method according to the invention can thus be employed advantageously for diagnosing disease conditions associated with altered, in particular elevated levels of TK1 activity.

A further aspect of the invention thus is an in vitro method for diagnosing and/or therapeutic monitoring of diseases in a human or animal involving elevated levels of thymidine kinase 1 activity comprising the steps of
a) obtaining a sample of human or animal body fluid or a cell or tissue sample
b) assaying the sample to determine the thymidine kinase 1 activity according to a method of the present invention; and
c) relating the amount of thymidine kinase 1 activity to the clinical status of the human or animal.

Diseases in a human or animal involving elevated levels of thymidine kinase 1 activity which may be mentioned by example in connection with the invention are cancer such as haematological cancer diseases, Non-Hodgkin's lymphoma, chronic lymphatic leukaemia, multiple myeloma, myelodysplastic syndrome, acute myelitic leukaemia, gastrointestinal cancer, prostate cancer and breast cancer.

The present invention also relates to kit for the in vitro diagnosis and/or therapeutic monitoring of diseases in a human or animal involving elevated levels of thymidine kinase 1 activity. Such kit will comprise AZT or a 3' derivative of thymidine. Depending on how the method is carried out, the kit may comprise other components. Other components which may be mentioned as examples are antibodies specific for the 5'-monophosphate of AZT or the 5'-monophosphate of the 3'-derivative of thymidine, buffer solutions, washing solutions, reagents which are required for the detection step, microtiter plates

Preferably all components of the kit are packed together in a container.

### References

1. Wilson, G.D., Assessment of human tumor proliferation using bromodeoxyuridne - current status. Acta Oncol., 30:903-910,1991.
2. Hall P.A, Woods A.L., Immunohistochemical markers of cellular proliferation: achievements, problems and prospects. Cell Tissue kinet. 23:505-522, 1990.
3. Bradshaw, H.D. Jr., Deininger, P.L., Human thymidine kinase gene: Molecular cloning and nucleotide sequence of a cDNA expressible in mammalian cells, Mol. Cell. Biol. 1984. 4, 2316-2320.
4. Sherley JL, Kelly TJ. Regulation of human thymidine kinase during the cell cycle. J Biol Chem. 1988 Jun 15;263(17):8350-8358.
5. Sherley JL, Kelly TJ. Human cytosolic thymidine kinase. Purification and physical characterization of the enzyme from HeLa cells. J Biol Chem. 1988 Jan 5;263(1):375-382.
6. Munch-Petersen B., Cloos G., Tyrsted G., Eriksson S. Diverging substrate specificity of pure human thymidine kinase 1 and 2 against antiviral dideoxy nucleosides., J. Biol Chem. 1991 266, 2032-22038.
7. Eriksson S, Kierdaszuk B, Munch-Petersen B, Oberg B, Johansson NG. Comparison of the substrate specificities of human thymidine kinase 1 and 2 and deoxycytidine kinase toward antiviral and cytostatic nucleoside analogs. Biochem Biophys Res Commun. 1991 Apr 30;176(2):586-92.
8. Furman PA, Fyfe JA, St Clair MH, Weinhold K, Rideout JL, Freeman GA, Lehrman SN, Bolognesi DP, Broder S, Mitsuya H, et al. Phosphorylation of 3'-azido-3'-deoxythymidine and selective interaction of the 5'-triphosphate with human immunodeficiency virus reverse transcriptase. Proc Natl Acad Sci U S A. 1986 Nov;83(21):8333-7.
9. Hampton, A., Chawla, R.R. and Kappler, F. Species or isozyme-specific enzyme inhibitors. 5. Differential effects of thymidine substituents on affinity for rat thymidine kinase isozymes. J. Med. Chem. 1982, 25, 644-649.
10. Lunato, J. A., Wang, J., Woollard, J. E., Anisuzzaman, A. B. K., Weihua, J., Rong, F-G., Ikeda, S., Soloway, A. H., Eriksson, S., Ives, D. H., Blue, T. E. and Tjarks, W. Synthesis of 5-(Carboranylalkylmercapto)-2'-deoxyuridines and 3-(Carboranylalkyl)thymidines and their evaluation as substrates for human thymidine kinase 1 and 2.. *J. Med. Chem*. 1999, 42, 3378-3389,
11. He Q., Skog S., Welander I., Tribukait B., Enzyme Kinetics of thymidine isoenzymes of Ehrlich ascites tumour cells. Cell prolif. 24, 3-14, 1991
12. Kaufmann M.G., Kelly T.J., Cell cycle regulation of thymidine kinase: Residues near the carboxyl terminus are essential for the specific degradation of the enzyme at mitosis., Mol. Cell Biol. 11: 2583-2546, 1991.
13. Wintersberger E. Regulation and biological function of thymidine kinase. Biochem Soc Trans. 1997 Feb;25(1):303-8.
14. Chang ZF, Huang DY, Chi LM. Serine 13 is the site of mitotic phosphorylation of human thymidine kinase. J Biol Chem. 1998 May 15;273(20):12095-100.
15. Wang L, Eriksson S. Cloning and characterization of full-length mouse thymidine kinase 2: the N-terminal sequence directs import of the precursor protein into mitochondria. Biochem J. 2000, 351, 469-76.
16. Saada A, Shaag A, Mandel H, Nevo Y, Eriksson S, Elpeleg O.Mutant mitochondrial thymidine kinase in mitochondrial DNA depletion myopathy. Nat Genet. 24, 342-344, 2001.
17. Tk-REA patent A method for determining dTk isoenzyme activity and the use therof, EP094923 priority 14.05.82 SE 82030401. EP filing date 10.05.83.
18. Hallek M., Langenmayer I., Nerl C., Knauf W., Dietzfelbinger H., Adorf D., Ostwald M., Busch R., Kuhn-Hallek I., Thiel E., Emmerich B. Elevated Serum Thymidine Kinase Levels Identify a subgroup at High Risk of Disease Progression in Early , Nonsmoldering Chronic Lymphocytic Leukaemia., Blood Vol 93, No5 (March 1) 1999: pp 1732-1737.
19. Di Raimondo, F., Giustolisi, R., Lerner, S., Cacciola, E., O'Brien, S., Kantarjian, H. and Keating, M.J. Retrospective study of the prognostic role of serum thymidine kinase level in CLL patients with active disease treated with fludarabine. Anals Oncol. 2001, 12, 621-625.
20. Broet P, Remain S, Daver A, Ricolleau G, Quillien V, Rallet A, Asselain B, Martin PM, Spyratos F.Thymidine kinase as a proliferative marker: clinical relevance in 1,692 primary breast cancer patients. J Clin Oncol. 2001 Jun 1;19(11):2778-87.
21. Foekens JA, Remain S, Look MP, Martin PM, Klijn JG. Thymidine kinase and thymidylate synthase in advanced breast cancer: response to tamoxifen and chemotherapy. Cancer Res. 2001 Feb 15;61(4):1421-5.
22. Remain S, Bendahl PO, Guirou O, Malmstrom P, Martin PM, Ferno M. DNA- synthesizing enzymes in breast cancer (thymidine kinase, thymidylate synthase and thymidylate kinase): association with flow cytometric S-phase fraction and relative prognostic importance in node-negative premenopausal patients. Int J Cancer. 2001 Jan 20;95(1):56-61.
23. Remain S, Spyratos F, Descotes F, Daver A, Rostaing-Puissant B, Bougnoux P, Colonna M, Bolla M, Martin. Prognostic of DNA-synthesizing enzyme activities (thymidine kinase and thymidylate synthase) in 908 T1-T2, N0-N1, M0 breast cancers: a retrospective multicenter study. Int J Cancer. 2000 Sep 15;87(6):860-8.
24. Span P, Heuvel J, Romain S, Piffanelli A, Martin PM, Geurts-Moespot A, Sweep EORTC receptor and biomarker study group report analytical and technical evaluation of a thymidine kinase radio-enzymatic assay in breast cancer cytosols. Anticancer Res. 2000 Mar-Apr;20(2A):681-7.
25. el Kouni, M. H., el Kouni, M. M., and Naguib, F. M. N. Differences in activities and substrate specificty of human and murine pyrimidine nucleoside phosphorylases: implications for chemotherapy with 5-fluoropyrimidines. Cancer Res. 1993, 53, 3687-3693.
26. Pauly, J.L., Schuller, M.G., Zelcer, A. A., Kriss, T.A. Gore, S.S. and Germain, M.J. Identification and comparative analysis of thymidine phosphorylase in plasma of healthy subsjects and cancer patients. J. Natl. Cancer Inst. 1977, 58, 1587-1590.
27. Shaw T, Smillie RH, MacPhee DG. The role of blood platelets in nucleoside metabolism: assay, cellular location and significance of thymidine phosphorylase in human blood. Mutat Res. 1988;200, 99-116
28. Balis M Earl. , et al. , USA patent 4,317,877 , Process for detecting the presences of malignant and pre malignant cells in humans., 2 March 1982
29. Jouan P. N., EP 0255431 A1 (Laboratories DEBAT), 3 Febr, 1988
30. O'Neill K., WO 9504758 A1 (Brigham Young University tech. Transfer off. , 16 Feb. 11,
31. Armstrong B., The development of a TK tumour marker system for cancer diagnosis, staging and treatment monitoring., Thesis submitted for the degree of doctor of philosophy, University of Ulster, 1991
32. He Q., Zou L., Zhang P.A, Lui J.X., Skog S., Fornander T., The clinical significance of thymidine kinase 1 measurement in serum of breast cancer patients using anti-TK1 antibody., Int. J. Biol. Markers 2000 Apr-Jun; 15 (2): 139-146
33. Kuroiwa N. , Nakayama M., Fukuda T., Fukui H., Ohwada H., Hiwasa T., Fujimura S., Specific recognition of cytosolic thymidine kinase in the human lung tumor by monoclonal antibodies raised against recombinant human thymidine Kinase., J immunol. Methods 2001 Jul 1; 253(1-2): 1-11
34. Zang F., Shao X., Li H., Robinson J. G., Murray B.K., O'Neill K.L., A monoclonal antibody specific for human thymidine kinase., Hybridoma 2001 feb; 20(1): 25-34
35. Zang F., Li H., Pendelton A.R., Robinson J.G., Monson K.O., Murray B.K., O'Neill K.L., Thymidine kinase 1 immunoassay: a potential marker for breast cancer., Cancer Detect Prev 2001; 25(1):8-15.
36. Karlström A.R., Neumüller M., Gronowitz J.S., Källander C.F.R., Molecular forms in human serum of enzymes synthesizing DNA precursors and DNA., Molecular and Cellular Biochemistry 92: 23-35, 1990
37. Lavie A., Schlichting I., Vetter I.R., Konrad M., Reinstein J., Goody R.S., The bottleneck in AZT activation., Nat Med 1997 Aug3(8):922-924
38. Tadepalli S.M., Quinn R.P., Determination of Zidovudine Concentrations in Serum by Enzyme-Linked Immunosorbent Assay and by Time-Resolved Fluoroimmunoassay., J. AIDS, 3:19-27, 1990
39. Goujon L., Brossette T., Dereudre-Brosquet N., Creminon C., Clayette P., Dormont D., Mioskowski C., Lebeau L., Grassi J. Monitoring of intracellular levels of 5'-monophosphate-AZT using an enzyme immunoassay,J. of Immunol. Methods 218 (1998) 19-30
40. Guettari N., Loubiere L., Brisson E., Klatzmann D. Use of herpes simplex virus thymidine kinase to improve the antiviral activity of zidovudine., Virology 1997 Sep 1; 235(2): 398-405
41. Suzutani T., Koyano S., Saijo M., Chiba A., Azuma M., Analysis of non-sense mutants of varicella-zoster virus thymidine kinase. Arch Virol 1997; 142(10):2059-64
42. Aslam M.and Dent A., *Bioconjugation;* Macmillan reference Ltd, 1998
43. Ellman. G.L., Tissue sulfhydrl groups; Arch. Biochem. Biophys, 82. 70-77; 1959
44. The Immunoassay Handbook, Edited by David Wild, The Macmillam Press Ltd. 1994.
45. Ellman G.L., Courtney K.D., Andres V., Featherstone R.M., A new colorimetric determination of acetylcholinesterase activity, Biochem. Pharmacol. 7, 88-95, 1961

## Claims

1. A method for determining thymidine kinase 1 activity in a human or animal body fluid or cell or tissue sample, comprising the steps of reacting said sample with a substrate for said thymidine kinase 1 which substrate is a 3'-derivative of thymidine in the presence of a phosphate donor and a buffer system and determining the amount of 5'-phosphorylated 3'-derivative of thymidine formed, said amount being related to said thymidine kinase 1 activity.

2. A method according to claim 1, wherein a substrate for TK1 is a 3'-deoxy-thymidine derivative of formula I 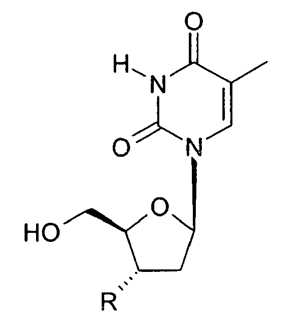 in which R is selected from but not limited to the group consisting of NH₂, NHCOCH₃, SC₂H₅, OC₂H₅, OBn, N₃, NO₂, OCOCH₃,OSO₂CH₃ and F.

3. A method according to claims 1 and 2, wherein the 3'-derivative of thymidine is AZT and the 5'-phosphorylated 3'-derivative of thymidine is AZTMP.

4. A method according to claims 1 to 3, wherein the amount of said 5'-phosphorylated 3'-derivative of thymidine formed is determined by an immunological method comprising reacting the 5'-phosphorylated 3'-derivative of thymidine formed with at least one antibody capable of selectively reacting with the 5'-phosphorylated 3'-derivative of thymidine to form immunocomplexes.

5. A method according to claim 4, wherein the amount of 5'-phosphorylated 3'-derivative of thymidine is determined by an immunological method using chemiluminescence.

6. A method according to claims 4 and 5, wherein the amount of said 5'-phosphorylated 3'-derivative of thymidine formed is determined by enzyme linked immunosorbent assay (ELISA).

7. A method according to claims 1 to 6 wherein said buffer comprises at least Dithioerythritol (DTE), ATP, MgCl₂ and HEPES or Tris and provides a pH from 6.5 to 8.0.

8. A method according to claims 1 to 6, wherein Uridine monophosphate (UMP) is contained in said buffer.

9. A method according to claims 1 to 6 wherein said substrate is present in a concentration of at least 0,4 µM.

10. A method according to claims 1 to 6 wherein said phosphate donor is present in a concentration of 0,1-10 mM.

11. Use of a method according to one of the forgoing claims for the diagnosis of diseases involving elevated levels of thymidine kinase 1 activity.

12. Use according to claim 11 for diagnosing cancer or tumours and for monitoring the progression of cancer or tumours.

13. Use according to claim 12 wherein cancer is selected from the group consisting of haematological cancer, breast cancer, gastrointestinal cancer and prostate cancer.

14. Use according to claim 11 for the identification of a subgroup of patients at high risk of disease progression in Non-Hodgkin's lymphoma and chronic lymphocytic leukaemia.

15. An in vitro method for diagnosing and/or therapeutic monitoring of diseases in a human or animal **characterised in** having elevated levels of thymidine kinase 1 activity comprising the steps of a) obtaining a sample of human or animal body fluid or a cell or tissue sample; b) assaying the sample to determine the thymidine kinase 1 activity according to a method of claims 1 to 10; and c) relating the amount of thymidine kinase 1 activity to the clinical status of the human or animal.

16. A kit for the in vitro diagnosis and/or therapeutic monitoring of diseases in a human or animal **characterised in** having elevated levels of thymidine kinase 1 activity comprising a) a 3'-derivative of thymidine; b) a phosphate donor; c) a buffer; and d) at least one antibody capable of selectively reacting with the 5'-phopshorylated 3'-derivative of thymidine.

17. A kit according to claim 16, wherein the 3'-derivative of thymidine is AZT and wherein the 5'-phopshorylated 3'-derivative of thymidine is AZTMP.

18. A kit according to claims 16 and 17 additionally comprising UMP.

19. A kit according to claim 16 to 18, wherein the reagents are packed together in a container.

## Patentansprüche

1. Verfahren zur Bestimmung der Thymidinkinase-1-Aktivität in einer menschlichen oder tierischen Körperflüssigkeits- oder Zell- oder Gewebeprobe umfassend die Schritte des Reagierens der Probe mit einem Substrat für die Thymidinkinase 1, wobei das Substrat ein 3'-Derivat des Thymidins in Anwesenheit eines Phosphatdonors und eines Puffersystems ist, und des Bestimmens der Menge an gebildetem 5'-phosphoryliertem 3'-Derivat des Thymidins, wobei die Menge sich auf die Thyminkinase-1-Aktivität bezieht.

2. Verfahren nach Anspruch 1, worin ein Substrat für TK-1 ein 3'-Desoxythymidinderivat der Formel I ist, 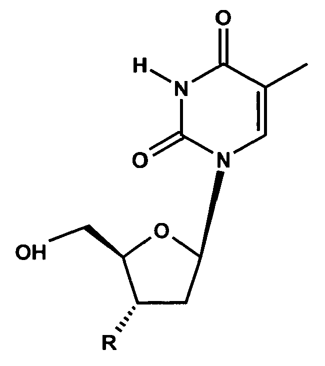 in welcher R ausgwählt aber nicht darauf beschränkt ist aus der Gruppe, welche NH₂, NHCOCH₃, SC₂H₅, OC₂H₅, OBn, N₃, NO₂, OCOCH₃, OSO₂CH₃ und F umfasst.

3. Verfahren nach den Ansprüchen 1 und 2, worin das 3'-Derivat des Thymindins AZT ist und das 5'-phosphorylierte 3'-Derivat des Thymidins AZTMP ist.

4. Verfahren nach den Ansprüchen 1 bis 3, worin die Menge an gebildetem 5'-phosphorylierten 3'-Derivat des Thymidins durch eine immunologische Methode bestimmt wird, welche umfasst das Reagieren des gebildeten 5'-phosphoryliertem 3'-Derivat des Thymidins mit mindestens einem zum selektiven Reagieren mit dem 5'-phosphorylierten 3'-Derivat des Thymindins geeigneten Antikörper, um einen Immunkomplex zu bilden.

5. Verfahren nach Anspruch 4, worin die Menge an 5'-phosphoryliertem 3'-Derivat des Thymidins bestimmt wird durch immunologische Methode unter Verwendung von Chemilumineszenz.

6. Verfahren nach den Ansprüchen 4 und 5, worin die Menge an gebildetem 5'-phosphoryliertem 3'-Derivat des Thymidins durch enyzmgekoppelte Immunadsorbtionsbestimmung (ELISA) bestimmt wird.

7. Verfahren nach den Ansprüchen 1 bis 6, worin der Puffer mindestens Dithioerythritol (DTE), ATP, MgCl2 und HEPES oder Tris umfasst und einen pH von 6,5 bis 8,0 zur Verfügung stellt.

8. Verfahren nach den Ansprüchen 1 bis 6, worin Uridinmonophosphat (UMP) in dem Puffer enthalten ist.

9. Verfahren nach den Ansprüchen 1 bis 6, worin das Substrat in einer Konzentration von mindestens 0,4 µM vorliegt.

10. Verfahren nach den Ansprüchen 1 bis 6, worin der Phosphatdonor in einer Konzentration von 0,1 bis 10 mM vorliegt.

11. Verwendung eines Verfahrens nach einem der voranstehenden Ansprüche zur Diagnose von Krankheiten, welche zu erhöhten Pegeln an Thymidinkinase-1-Aktivität führen.

12. Verwendung nach Anspruch 11 zur Diagnose von Krebs oder Tumoren und zur Überwachung des Verlaufs von Krebs oder Tumoren.

13. Verwendung nach Anspruch 12, worin der Krebs ausgewählt ist aus der Gruppe, welche Blutkrebs, Brustkrebs, gastroindestinal Krebs und Prostatakrebs umfasst.

14. Verwendung nach Anspruch 11 zur Identifizierung einer Untergruppe von Patienten mit hohem Risiko des Krankeitsverlaufs bei nicht-Hodgkin Lymphom und chronischer lymphozytischer Leukämie.

15. In-vitro-Methode zur Diagnose und/oder therapeutsichen Überwachung von Krankheiten in einem Menschen oder Tier, welche durch Aufweisen von erhöhten Pegeln der Thymidinkinase-1-Aktivität gekennzeichnet sind, umfassend die Schritte a) Erhalten einer Probe an menschlicher oder tierischer Körperflüssigkeit oder einer Zelle oder Gewebeprobe, b) Untersuchen der Probe, um die Thymidinkinase-1-Aktivität nach einer Methode der Ansprüche 1 bis 10 zu bestimmen, c) Zuordnen der Menge an Thymidinkinase-1-Aktivität auf den klinischen Zustand des Menschen oder Tieres.

16. Ausrüstung zur *in-vitro*-Diagnose und/oder therapeutischen Überwachung von Krankheiten in einem Menschen oder Tier, welche durch Aufweisen von erhöhten Pegeln der Thymidinkinase-1-Aktivität gekennzeichnet sind, umfassend a) ein 3'-Derivat des Thymidins, b) einen Phosphatdonor, c) einen Puffer und d) mindenstens einen zum selektiven Reagieren mit dem 5'-phosphorylierten 3'-Derivat des Thymidins geeigneten Antikörper.

17. Ausrüstung nach Anspruch 16, worin das 3'-Derivat des Thymidins AZT ist und worin das 5'-phosphorylierte 3'-Derivat des Thymidins AZTMP ist.

18. Ausrüstung nach den Ansprüchen 16 und 17 zusätzlich umfassend UMP.

19. Ausrüstung nach Anspruch 16 bis 18, worin die Reagenzien zusammen in einen Behälter gepackt sind.

## Revendications

1. Procédé de détermination de l'activité de la thymidine kinase 1 dans un fluide biologique, une cellule ou un échantillon de tissu humain ou animal, comprenant la mise en réaction dudit échantillon avec un substrat pour ladite thymidine kinase 1, lequel substrat est un dérivé 3' de thymidine en présence d'un donneur de phosphate et d'un système tampon et la détermination de la quantité de dérivé 3' à extrémité 5' phosphorylée de thymidine formé, ladite quantité étant liée à ladite activité de la thymidine kinase 1.

2. Procédé selon la revendication 1, dans lequel un substrat pour TK1 est un dérivé 3'-désoxy-thymidine de formule 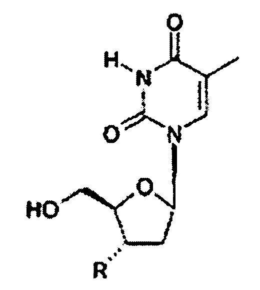 dans laquelle R est choisi, entre autres, dans le groupe constitué par NH₂, NHCOCH₃, SC₂H₅, OC₂H₅, OBn, N₃, NO₂, OCOCH₃, OSO₂CH₃ et F.

3. Procédé selon les revendications 1 et 2, dans lequel le dérivé 3' de thymidine est AZT et le dérivé 3' à extrémité 5' phosphorylée de thymidine est AZTMP.

4. Procédé selon les revendications 1 à 3, dans lequel la quantité dudit dérivé 3' à extrémité 5' phosphorylée de thymidine formé est déterminée par le biais d'un procédé immunologique comprenant la mise en réaction du dérivé 3' à extrémité 5' phosphorylée de thymidine formé avec au moins un anticorps capable de réagir de manière sélective avec le dérivé 3' à extrémité 5' phosphorylée de thymidine pour former des immunocomplexes.

5. Procédé selon la revendication 4, dans lequel la quantité de dérivé 3' à extrémité 5' phosphorylée de thymidine est déterminée par le biais d'un procédé immunologique utilisant la chimiluminescence.

6. Procédé selon les revendications 4 et 5, dans lequel la quantité dudit dérivé 3' à extrémité 5' phosphorylée de thymidine formé est déterminée par le biais d'un dosage par immunosorbant lié à une enzyme (ELISA).

7. Procédé selon les revendications 1 à 6, dans lequel ledit tampon comprend au moins du dithioérythritol (DTE), de l'ATP, du MgCl₂ et du HEPES ou du Tris et confère un pH compris entre 6,5 et 8,0.

8. Procédé selon les revendications 1 à 6, dans lequel de l'uridine monophosphate (UMP) est contenue dans ledit tampon.

9. Procédé selon les revendications 1 à 6, dans lequel ledit substrat est présent dans une concentration d'au moins 0,4 µM.

10. Procédé selon les revendications 1 à 6, dans lequel ledit donneur de phosphate est présent dans une concentration comprise entre 0,1 et 10 mM.

11. Utilisation d'un procédé selon l'une quelconque des revendications précédentes, pour le diagnostic de maladies impliquant des niveaux élevés d'activité de la thymidine kinase 1.

12. Utilisation selon la revendication 11, pour diagnostiquer le cancer ou les tumeurs et pour surveiller l'évolution du cancer ou des tumeurs.

13. Utilisation selon la revendication 12, dans laquelle le cancer est choisi dans le groupe constitué par le cancer hématologique, le cancer du sein, le cancer gastro-intestinal et le cancer de la prostate.

14. Utilisation selon la revendication 11, pour l'identification d'un sous-groupe de patients à haut risque d'évolution de la maladie dans le lymphome non hodgkinien et la leucémie lymphocytaire chronique.

15. Procédé in vitro de diagnostic et/ou de surveillance thérapeutique de maladies chez un humain ou un animal **caractérisé en ce qu'**il présente des niveaux élevés d'activité de la thymidine kinase 1, comprenant les étapes consistant a) obtenir un échantillon de fluide biologique ou un échantillon de cellule ou de tissu humain ou animal ; b) évaluer l'échantillon pour déterminer l'activité de la thymidine kinase 1 selon un procédé des revendications 1 à 10 ; et c) mettre e relation la quantité d'activité de la thymidine kinase 1 avec l'état clinique de l'humain ou de l'animal.

16. Kit de diagnostic in vitro et/ou de surveillance thérapeutique de maladies chez un humain ou un animal **caractérisé en ce qu'**il présente des niveaux élevés d'activité de la thymidine kinase 1 comprenant a) un dérivé 3' de thymidine ; b) un donneur de phosphate ; c) un tampon; et d) au moins un anticorps capable de réagir de manière sélective avec le dérivé 3' à extrémité 5' phosphorylée de thymidine.

17. Kit selon la revendication 16, dans lequel le dérivé 3' de thymidine est AZT et dans lequel le dérivé 3' à extrémité 5' phosphorylée de thymidine est AZTMP.

18. Kit selon les revendications 16 et 17, comprenant en plus de l'UMP.

19. Kit selon les revendications 16 à 18, dans lequel les réactifs sont emballés ensemble dans un récipient.
